Europäisches Patentamt

European Patent Office

Office européen des brevets

(11) Veröffentlichungsnummer: **0 007 461**

**A1**

(12) **EUROPÄISCHE PATENTANMELDUNG**

(21) Anmeldenummer: **79102187.6**

(22) Anmeldetag: **29.06.79**

(51) Int. Cl.³: **C 12 N 7/02**
**//A61K39/29, A61K39/42,**
**G01N33/54**

(30) Priorität: **06.07.78 DE 2829741**

(43) Veröffentlichungstag der Anmeldung:
**06.02.80 Patentblatt 80/3**

(84) Benannte Vertragsstaaten:
**AT BE CH DE FR GB IT LU NL SE**

(71) Anmelder: **BEHRINGWERKE AKTIENGESELLSCHAFT**
**Postfach 1140**
**D-3550 Marburg/Lahn(DE)**

(72) Erfinder: **Lorenz, Peter Rolf, Dr.**
**Georg-Speyer-Strasse 79**
**D-6000 Frankfurt am Main 90(DE)**

(72) Erfinder: **Weinmann, Ernst, Dr.**
**Sonnenweg 19**
**D-3550 Marburg/Lahn(DE)**

(72) Erfinder: **Majer, Mirko, Dr.**
**Mozartstrasse 5**
**D-8510 Fürth(DE)**

(74) Vertreter: **Meyer-Dulheuer, Karl-Hermann, Dr. et al,**
**HOECHST Aktiengesellschaft Zentrale Patentabteilung**
**Postfach 80 03 20**
**D-6230 Frankfurt/Main 80(DE)**

(54) Verfahren zur Gewinnung des Hepatitis A-Virus.

(57) Zur Gewinnung des Hepatitis A-Virus werden Affen mit Hepatitis A-Virus infiziert und das Hepatitis A-Virus aus dem Stuhl der Affen isolier.

EP 0 007 461 A1

Croydon Printing Company Ltd

- 1 -

BEHRINGWERKE AKTIENGESELLSCHAFT    HOE 78/B 006   Dr.LI/sch

<u>Verfahren zur Gewinnung des Hepatitis A-Virus</u>

Gegenstand der Erfindung ist ein Verfahren zur Gewinnung des Hepatitis A-Virus, das Träger des spezifischen Hepatitis A-Antigens ist.

Der Erreger der viralen Leberentzündung, das Hepatitis A-Virus, wird gegenwärtig aus dem Stuhl erkrankter Personen gewonnen. Die Methode hat den Nachteil, daß man für die Gewinnung des Rohmaterials von einem großen Patientenkries, der z.B. bei Ausbruch einer Hepatitis A-Epidemie anfällt, abhängig ist.

Außerdem wird das Hepatitis A-Virus aus den Lebern von Krallenäffchen und Menschenaffen der Arten Sanguinus (S.) mystax, S.nigricollis, S.fuscicollis, S.oedipus, Callithrix (C.)jacchus, C.argentata, Cercopithecus aethiops, Pan troglodytes und Anthropopithecus troglodytes gewonnen. Alle genannten Arten sind jedoch vom Aussterben bedroht und im allgemeinen nur mit großem Aufwand und geringem Erfolg züchtbar.

Um das Hepatitis A-Virus aus den Lebern isolieren zu können, müssen die Spendertiere geopfert werden.

In Gewebekulturen ist das Hepatitis A-Virus nach dem derzeitigen Stand der wissenschaftlichen Erkenntnisse nicht züchtbar.

- 2 -

Es wurde nun ein Verfahren gefunden, das dadurch gekennzeichnet ist, daß Affen insbesondere solche der Familie der Callithriden vorzugsweise Exemplare der Art Callithrix jacchus mit Hepatitis A-Virus infiziert und das Hepatitis A-Virus aus dem Stuhl der Affen isoliert wird.

Das Verfahren ist außerdem dadurch gekennzeichnet, daß die Tiere mit Immunsuppressiva behandelt werden.

Man geht zweckmäßigerweise so vor, daß den Tieren einige, insbesondere dreiTage vor der Infektion mit Hepatitis A-Virus bis etwa 9 Tage nach der Infektion ein Immunsuppressivum gegeben wird. Als Immunsuppressiva im Sinne der Erfindung kommen sowohl solche infrage, die die humorale Immunität unterdrücken als auch solche, die die zelluläre Immunität unterdrücken. Als Beispiel der zuerst genannten Gruppe sei Cortison, als Beispiel der zuletztgenannten Cyclophosphamid erwähnt. Es können aber auch Immunsuppressiva verwendet werden, die keiner dieser Gruppen zuzuordnen sind wie Antilymphozyten-Globulin oder andere Maßnahmen der Unterdrückung der Immunreaktionen beispielsweise Bestrahlung.

Es hat sich als günstig erwiesen, täglich ein Immunsuppressivum zu geben und zwischen verschieden wirkenden Immumsupressiva abzuwechseln. In einer bevorzugten Ausführungsform erhalten die infizierten Affen abwechselnd einen Tag Cortison und einen Tag Cyclophosphamid. Das Immunsuppressivum wird einmal täglich verabreicht. Im Bedarfsfall kann die Verabreichung auch in größeren Abständen, z.B. alle 2 Tage erfolgen. Die Dosis soll an der oberen Grenze der Verträglichkeit liegen, diese beträgt für Cortison etwa 5 mg/kg und für Cyclophosphamid etwa 10 mg/kg.

Die Wirkung der Immunsuppressiva wird durch Zählen der weißen Blutkörperchen kontrolliert. Normalerweise sind 4.000 bis 7.500 weiße Blutkörperchen in einem $mm^3$ Blut enthalten. Durch die Immunsuppressiva nehmen die weißen Blutkörperchen bis etwa 2.500 ab. Sind sie unter 2.000 gefallen, wird die Immunsuppression abgebrochen.

Etwa 30 Tagelang beginnend mit dem Tag der Infektion, wird der Stuhl der Tiere gesammelt, bei -20°C gelagert um später daraus das Hepatitis A-Virus zu isolieren. Die Tiere brauchen nicht getötet zu werden und können nach Ausheilen der Infektion für die Zucht oder für die Gewinnung von Hepatitis A-Antikörper verwendet werden.

Zur Isolierung des Hepatitis A-Virus aus dem Stuhl der Affen werden die täglichen Stuhlproben auf Vorhandensein von Hepatitis A-Virus immunologisch untersucht, z.B. mittels Radioimmunassay oder Immunoperoxydase-Test. Hierzu werden geringe Mengen - etwa 100 mg - der Stuhlproben mit einer 9-fachen Menge - auf Gewicht bezogen - von physiologischer Kochsalzlösung aufgeschwemmt, 15 Minuten auf einem Mischer stark geschüttelt und klar zentrifugiert. Der Überstand wird für die Untersuchung auf Hepatitis A-Virus verwendet.

Die positiv gefundenen Stuhlproben werden in physiologischer Kochsalzlösung (etwa der doppelten bis dreifachen Gewichtsmenge) aufgeschwemmt gut durchmischt und nach einiger Zeit (etwa 10 - 60 Minuten) der flüssige Überstand von den Feststoffteilen abgetrennt. Der resultierende Überstand enthält das Hepatitis-A-Virus. Er kann gewünschtenfalls konzentriert werden.

Aus der gewonnenen Menge Hepatitis A-Virus wurde ermittelt, daß die von einem unter Immunsuppression infizierten Versuchstier gewonnene Hepatitis A-Virus-haltige Stuhlmenge für die Herstellung von 5.000 Testen ausreicht. In einem Infektionsversuch ohne Immunsuppression ist der Hepatitis A-Virusgehalt des Stuhles deutlich geringer.

Das isolierte Hepatitis A-Virus dient als diagnostisches Reagenz zum Nachweis von Antikörpern gegen Hepatitis A-Virus in Patienten mit Leberentzündungen. Es kann auch zu einem Impfstoff aufgearbeitet und zur Gewinnung von

- 4 -

Hepatitis A-Antikörpern gegen Hepatitis A-Virus verwendet werden. Auch zur Durchführung des erfindungsgemäßen Verfahrens kann es benutzt werden.

Beispiel

Zwei Callithrix jacchus-Äffchen werden 12 Tage mit Immunsuppressiva behandelt, am 1. und allen folgenden ungeraden Tagen mit Cortison, am 2. und an allen folgenden geraden Tagen bis zum 12. Tag mit Cyclophosphamid. Am 3. Tage werden die Äffchen durch i.v. Injektion von einem Milliliter einer Aufschwemmung von Hepatitis A-Virus, das aus dem Stuhl von Hepatitis-Patienten gewonnen wurde, in die Oberschenkelvene infiziert. Die Stuhlproben der Tiere werden bis zum 30. Tag nach der Infektion täglich bei -20°C eingefroren und aufbewahrt. Danach wird in den täglichen Stuhlproben der Gehalt an Hepatitis A-Virus geprüft, die positiven Stühle zusammengegeben (73,5 g) und mit der doppelten Gewichtsmenge physiologischer Kochsalzlösung aufgeschwemmt. Anschließend wird 15 Minuten auf einem Mischer geschüttelt und klar zentrifugiert. Der resultierende Überstand reicht für etwa 10.000 Teste auf Hepatitis A-Antikörper.

0007461

**Patentansprüche:**

1. Verfahren zur Gewinnung des Hepatitis A-Virus, dadurch gekennzeichnet, daß Affen mit Hepatitis A-Virus infiziert werden und das Hepatitis A-Virus aus dem Stuhl der Affen isoliert wird.

2. Verfahren nach Anspruch 1, dadurch gekennzeichnet, daß Affen der Familie der Callithiciden verwendet werden.

3. Verfahren nach Anspruch 2, dadurch gekennzeichnet, daß Affen der Art Callithrix jacchus verwendet werden.

4. Verfahren gemäß Anspruch 1, dadurch gekennzeichnet, daß die Affen mit Immunsuppressiva behandelt werden.

Europäisches
Patentamt

**EUROPÄISCHER RECHERCHENBERICHT**

Nummer der Anmeldung

**EP 79 10 2187**

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | CHEMICAL ABSTRACTS, vol. 87, Nr. 19, November 7, 1977, abstract 149715r, Seite 386. Columbus, Ohio, USA BRADLEY,D.W. et al. "Cyclic excretion of hepatitis A virus in experimentally infected chimpanzees: biophysical characterization of the associated HAV particles". & J. MED. VIROL. 1977, 1(2), 133-8. * Zusammenfassung * -- | 1-4 |
| | CHEMICAL ABSTRACTS, vol. 87, Nr. 11, September 12, 1977, abstract 82978h, Seite 437. Columbus, Ohio, USA BRADLEY, D.W. et al."Purification of hepatitis A virus from chimpanzee stools". & J. VIROL. 1977 22(1), 228-31. * Zusammenfassung * -- | 1-4 |
| | MAYR A., BACHMANN P.A., BIBRACK B. und WITTMANN G.: "Virologische Arbeitsmethoden", Band I, Zellkulturen-Bebrütete Hühnereier-Versuchstiere. VEB Gustav Fischer Verlag, Jena, 1974. * Seite 561, Abschitt 3.1.5.1 * -- | 1-4 |
| P | FR - A - 2 398 504 (INSTITUT DE VIROLOGIE DE TOURS) * Seite 1, Zeilen 17-20 * ---- | 1-4 |

**KLASSIFIKATION DER ANMELDUNG** (Int.Cl. 3)

C 12 N 7/02//
A 61 K 39/29
        39/42
G 01 N 33/54

**RECHERCHIERTE SACHGEBIETE** (Int. Cl. 3)

C 12 N 7/00
A 61 K 39/29

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt

| Recherchenort | Abschlußdatum der Recherche | Prüfer |
|---|---|---|
| Den Haag | 17-10-1979 | RYCKEBOSCH |

EPA form 1503.1 06.78